# EUROPÄISCHE PATENTSCHRIFT

(11) **EP 0 982 280 B1**
(45) Veröffentlichungstag und Bekanntmachung des Hinweises auf die Patenterteilung: **05.06.2002**
(21) Anmeldenummer: 99115194.5
(22) Anmeldetag: 16.08.1999
(51) Int. Cl.: C07C 11/16, C07C 7/04

(54) **Verfahren zur Gewinnung von Butadien-1,2**
Process for the recovery of butadiene-1,2
Procédé pour la récupération de butadiène-1,2

(30) Priorität: 27.08.1998 DE 19838932
(43) Veröffentlichungstag der Anmeldung: 01.03.2000
(73) Patentinhaber: BP Köln GmbH, D-50769 Köln (DE)
(72) Erfinder: Stüwe, Arnd, Dr., 51373 Leverkusen (DE); Linnemann, Jürgen, Dr., 41470 Neuss (DE); Herwig, Jens, Dr., 50859 Köln (DE); Gabel, Christian, Dr., 41539 Dormagen (DE); Hohmann, Bernd, 41539 Dormagen (DE); Grub, Joachim, Dr., 41539 Dormagen (DE)
(74) Vertreter: Weber, Thomas, Dr.Dipl.-Chem.

(56) Entgegenhaltungen:
- DD-A- 246 009
- DE-A- 2 331 547

## Beschreibung

Die vorliegende Erfindung betrifft ein Verfahren zur Gewinnung von Butadien-1,2 aus einer Polymerisationsinhibitor-haltigen C₄-Kohlenwasserstofffraktion durch fraktionierte Destillation.

Die Isolierung von Butadien-1,3 aus einem Gemisch von C₄-Kohlenwasserstoffen ist durch einfache Destillation nicht möglich, da alle Komponenten in einem sehr engen Temperaturbereich sieden und einige darüber hinaus azeotrope Gemische bilden. Aus diesem Grund wird Butadien-1,3 heutzutage großtechnisch nach dem Prinzip der Extraktivdestillation gewonnen. Bei diesem Verfahren wird einem gasförmigen C₄-Kohlenwasserstoffgemisch aus der Naphtha- oder Mitteldestillat-Pyrolyse in einer Extraktionskolonne ein Lösungsmittel zugeführt. Dieses Lösungsmittel löst vor allem Butadien-1,3, welches dadurch selektiv extrahiert wird. Das Butadien-1,3 enthaltende Lösungsmittel verbleibt somit im Sumpf der Kolonne, während die restliche C₄-Fraktion über Kopf abdestilliert. Als Lösungsmittel werden beispielsweise Sulfolan, N-Methylpyrrolidon (NMP), Dimethylformamid, Acetonitril oder Dimethylacetamid eingesetzt. Um die unerwünschte thermische Polymerisation des Butadien-1,3 im Verlauf der Extraktivdestillation zu vermeiden, setzt man sowohl der Einsatzfraktion der Extraktivdestillation als auch dem Sumpfprodukt aus Lösungsmittel und Butadien-1,3 Polymerisationsinhibitoren zu. Hierbei handelt es sich beispielsweise um 4-tert. Butylbrenzcatechin (TBC). Im Verlauf der nachfolgenden Trennoperationen zur Reingewinnung des Butadien-1,3 fallen daher Destillationrückstände von C₄- und C₅-Kohlenwasserstoffen an, die diese Polymerisationsinhibitoren in zum Teil erheblichen Mengen enthalten. Es ist allgemein üblich, derartige Destillationsrückstände bzw. Sumpfprodukte aus der Feinreinigung des Butadien-1,3 zu vernichten. Dies geschieht in der Regel durch Verbrennung über eine Fackel oder durch sonstige thermische Verwertung. Bei dieser Verfahrensweise gehen wertvolle Kohlenwasserstoffe, die zur stofflichen Nutzung geeignet sind, verloren.

Aus DD 246 009 ist ein Verfahren bekannt zur Aufarbeitung von solchen Destillationsrückständen, die bei der Extraktivdestillation von C₄-Kohlenwasserstofffraktionen zur Gewinnung von Butadien-1,3 anfallen und gelöste Polymerisationsinhibitoren enthalten. Bei diesem Verfahren wird das Inhibitor/C₄-Kohlenwasserstoffgemisch zunächst in ein vorzugsweise Aromaten enthaltendes Kohlenwasserstoffgemisch eingebracht, dessen Siedebeginn 50 - 200 K höher liegt als der Siedepunkt der C₄-Kohlenwasserstofffraktion, und anschließend thermisch behandelt. Hierbei wird die Temperatur so eingestellt, daß die C₄-Kohlenwasserstofffraktion verdampft und damit vollständig entfernt werden kann. Im Sumpf der Kolonne verbleibt entsprechend ein Gemisch aus den höhersiedenden, vorzugsweise aromatischen Kohlenwasserstoffen, insbesondere C₈- und C₉-Aromaten, den Schwersiedern, den Verunreinigungen sowie dem Polymerisationsinhibitor. Dieses Verfahren ermöglicht somit die Abtrennung der C₄-Kohlenwasserstofffraktion als solcher von den Verunreinigungen und Schwersiedern sowie insbesondere dem Inhibitor. Die gesamte C₄-Kohlenwasserstofffraktion wird einer stofflichen oder kalorischen Verwertung zugeführt; eine weitere Auftrennung in die verschiedenen Komponenten wird nicht beschrieben.

Eine Gewinnung der Einzelverbindungen aus solchen C₄-Kohlenwasserstofffraktionen ist jedoch wünschenswert. Vor allem die C₄-Komponente Butadien-1,2 gewinnt zunehmend an Bedeutung und wird als Polymerisationsregulator bei der Herstellung von Synthesekautschuk aus Butadien-1,3 eingesetzt. Auch für die Riechstoffherstellung stellt Butadien-1,2 einen interessanten Synthesebaustein dar. So liefert die Umsetzung von Butadien-1,2 mit Acetaldehyd cis-3-Hexenol, den sogenannten Blätteralkohol (Leaf alcohol).

Aus DE-A-2 331 547 ist bereits ein Verfahren zur Gewinnung und Reinigung von Butadien-1,2 bekannt. In diesem Verfahren wird in einer ersten Stufe aus einem Butadien-1,3-Gewinnungsverfahren eine hochsiedende Kohlenwasserstofflösung erhalten, die ungesättigte und im Wesentlichen C₄-Kohlenwasserstoffe enthält, in einer zweiten Stufe werden diese ungesättigten im Wesentlichen C₄-Kohlenwasserstoffe von dem hochsiedenden Kohlenwasserstoff abgetrennt und in einer dritten Stufe wird ein wenigstens 80 % Butadien-1,2 enthaltender Butadien-1,2-Strom aus den ungesättigten, im Wesentlichen C₄-Kohlenwasserstoffe, gewonnen.

Die Aufgabe der vorliegenden Erfindung bestand somit darin, ein Verfahren bereitzustellen, welches die Gewinnung von reinem Butadien-1,2 in einfacher Weise aus C₄-Kohlenwasserstofffraktionen ermöglicht.

Gelöst wird diese Aufgabe durch ein Verfahren zur Gewinnung von Butadien-1,2, bei dem eine Polymerisationsinhibitor-haltige C₄-Kohlenwasserstofffraktion mindestens einer fraktionierten Destillation unterworfen wird.

Es ist überraschend, daß die Abtrennung und Gewinnung von reinem Butadien-1,2 aus C₄-Kohlenwasserstoffgemischen ohne vorhergehende Abtrennung des Polymerisationsinhibitors und ohne sonstige Zusätze anderer höhersiedender Kohlenwasserstofffraktionen gelingt. Aufgrund eines erheblichen Gehalts an Inhibitor im zu destillierenden Ausgangsgemisch und der weiteren Aufkonzentrierung dieses Inhibitors im Verlauf der fraktionierten Destillation war zu erwarten, daß der Inhibitor zunehmend auskristallieren würde, da die Löslichkeit von Inhibitoren in Kohlenwasserstoffgemischen nur sehr gering ist. In Isopentan löst sich der Polymerisationsinhibitor TBC bei 20°C beispielsweise nur zu 0.5 %. Ein solches Auskristallisieren des Inhibitors sollte zu einer Belegung der Destillationskolonne führen, was Verstopfungen der Kolonne und damit eine Verschlechterung der Trennleistung nach sich ziehen würde. Unerwarteterweise tritt jedoch keines dieser Phänomene im erfindungsgemäßen Verfahren auf.

Der Einsatzstrom für das erfindungsgemäße Verfahren wird üblicherweise erhalten, indem man aus dem Sumpf oder an einem geeigneten Boden im Abtriebsteil einer Destillationskolonne, bei der am Kopf Butadien-1,3 als Reinprodukt gewonnen wird, die Polymerisationsinhibitor-haltige C₄-Kohlenwasserstofffraktion entnimmt.

Diese Polymerisationsinhibitor-haltige C₄-Kohlenwasserstofffraktion weist einen Siedebereich von -15°C bis +45°C auf. Sie enthält neben leichtsiedenden C₄-Komponenten, wie z.B. Butanen, Butadien-1,3, Butenen und C₄-Acetylenen auch den gewünschten Wertstoff Butadien-1,2. Als hochsiedende Verbindungen findet man in der C₄-Kohlenwasserstofffraktion C₅-Kohlenwasserstoffe wie z.B. 3-Methyl-buten-1 und Isopentan. Weiterhin sind in dem C₄-Kohlenwasserstoffgemisch immer Polymerisationsinhibitoren wie das bereits erwähnte TBC enthalten. Je nach Fahrweise der Destillation zur Reingewinnung des Butadien-1,3 schwanken die Konzentrationen der einzelnen Komponenten im C₄-Kohlenwasserstoffgemisch in einem weiten Bereich. Üblicherweise sind in dem Polymerisationsinhibitor-haltigen C₄-Kohlenwasserstoffgemisch

| | |
|---|---|
| 0-5 Gew.% | gesättigte C₄-Kohlenwasserstoffe, |
| 5-30 Gew.% | Butene, |
| 10-55 Gew.% | Butadien-1,3, |
| 0,1-2 Gew.% | C₄-Acetylene, |
| 20-65 Gew.% | Butadien-1,2, |
| 5-20 Gew.% | C₅-Kohlenwasserstoffe sowie |
| 0.2-2 Gew.% | Polymerisationsinhibitor |

enthalten. Ein davon abweichender Anteil an Leichtsiedern bzw. Hochsiedern stört im erfindungsgemäße Verfahren nicht, macht aber gegebenenfalls Anpassungen der Destillationsbedingungen (Temperatur, Rücklaufverhältnis) und der Destillationseinrichtungen (Kolonnendurchmesser, theoretische Trennstufenzahl) erforderlich. Bevorzugt kommen im erfindungsgemäßen Verfahren C₄-Kohlenwasserstoffgemische zum Einsatz, die

| | |
|---|---|
| 0 - 1 Gew.% | gesättigte C₄-Kohlenwasserstoffe, |
| 10-20 Gew.% | Butene, |
| 20-55 Gew.% | Butadien-1,3, |
| 0.1-1 Gew.% | C₄-Acetylene, |
| 30-65 Gew.% | Butadien-1,2, |
| 5-10 Gew.% | C₅-Kohlenwasserstoffe sowie |
| 0.2-1 Gew.% | Polymerisationsinhibitor |

enthalten.

Gemäß einer Ausführungsform des erfindungsgemäßen Verfahrens wird so vorgegangen, daß man
a) in einer ersten fraktionierten Destillation der Polymerisationsinhibitor-haltigen C₄-Kohlenwasserstofffraktion als erstes Kopfprodukt die leichtsiedenden C₄-Kohlenwasserstoffe und als erstes Sumpfprodukt eine Fraktion, die Butadien-1,2, die C₅-Kohlenwasserstoffe und den Polymerisationsinhibitor enthält, entnimmt, und
b) das erste Sumpfprodukt einer zweiten fraktionierten Destillation zuführt und dort als zweites Kopfprodukt das Butadien-1,2 gewinnt und als zweites Sumpfprodukt die C₅-Kohlenwasserstoffe und den Polymerisationsinhibitor erhält.

Bei dieser Ausführungsform enthält das erste Kopfprodukt die leichtsiedenden C₄-Kohlenwasserstoffe wie Butadien-1,3 und die Butene. Das erste Sumpfprodukt, welches das gewünschte Butadien-1,2, die C₅-Kohlenwasserstoffe und den Polymerisationsinhibitor enthält, ist nahezu frei von den leichtsiedenden C₄-Kohlenwasserstoffen. Bei dem zweiten Kopfprodukt handelt es sich um das gewünschte Butadien-1,2 in einer Reinheit von mindestens 97 %, bevorzugt mindestens 99 %. Das zweite Sumpfprodukt kann neben den C₅-Kohlenwasserstoffen und der gesamten Menge des Polymerisationsinhibitors auch noch Restmengen an Butadien-1,2 enthalten. Aus diesem zweiten Sumpfprodukt kann der Polymerisationsinhibitor durch einen weiteren Destillationsschritt entfernt werden. Die dabei abgetrennten C₅-Kohlenwasserstoffe können dann einer stofflichen oder thermischen Verwertung zugeführt werden. Alternativ dazu ist es auch möglich, das zweite Sumpfprodukt ohne Abtrennung des Polymerisationsinhibitors direkt der thermischen Verwertung zuzuführen.

Die Ausbeute an Butadien-1,2, bezogen auf das in der eingesetzten C₄-Kohlenwasserstofffraktion enthaltene Butadien-1,2, beträgt bei dieser Ausführungsform mindestens 85 %, bevorzugt mindestens 87 % und insbesondere mindestens 90 %.

Die Ausbeute kann dabei noch weiter gesteigert werden, wenn man die Trennleistung der 2. Destillationskolonne verbessert, indem man beispielsweise die Zahl der theoretischen Böden erhöht, den Rücklauf vergrößert oder die Reinheitsansprüche für das Kopfprodukt Butadien-1,2 reduziert.

Die Destillationskolonnen können in beiden fraktionierten Destillationen mit Böden, Füllkörpern oder strukturierten Packungen ausgestattet sein. Beide fraktionierte Destillationen werden üblicherweise bei einem Druck von 0.1-1 MPa, bevorzugt 0.2-0.8 MPa und den sich bei diesem Druck einstellenden Siedetemperaturen durchgeführt.

In einer zweiten Ausführungsform des erfindungsgemäßen Verfahrens geht man so vor, daß man
a) in einer ersten fraktionierten Destillation der Polymerisationsinhibitor-haltigen C₄-Kohlenwasserstofffraktion als erstes Kopfprodukt die leichtsiedenden C₄-Kohlenwasserstoffe und Butadien-1,2 und als erstes Sumpfprodukt die C₅-Kohlenwasserstoffe und den Polymerisationsinhibitor entnimmt und
b) das erste Kopfprodukt in eine zweite fraktionierte Destillation einbringt und dort als zweites Kopfprodukt die leichtsiedenden C₄-Kohlenwasserstoffe und als zweites Sumpfprodukt das Butadien-1,2 gewinnt.

Das erste Sumpfprodukt enthält in diesem Fall neben den C₅-Kohlenwasserstoffen und der gesamten Menge des Polymerisationsinhibitors gegebenenfalls auch noch Restmengen an Butadien-1,2. Das erste Kopfprodukt wird üblicherweise nach der Entnahme zunächst in einem Kondensator verflüssigt und dann in die zweite fraktionierte Destillation eingebracht.

Gegebenenfalls ist es bei dieser Ausführungsform vorteilhaft, das Butadien-1,2 bei der zweiten fraktionierten Destillation nicht über das zweite Sumpfprodukt zu gewinnen, sondern in der zweiten Destillationskolonne bereits als Seitenstrom direkt oberhalb des Sumpfes, bevorzugt einige Böden oberhalb des Sumpfes zu entnehmen. Durch diese Variante ist es möglich, besonders reines Butadien-1,2 zu gewinnen.

Auch bei dieser zweiten Ausführungsform des erfindungsgemäßen Verfahrens können die Destillationskolonnen der beiden fraktionierten Destillationen als Einbauten Böden, Füllkörper oder strukturierte Packungen aufweisen.

Die Ausbeute an Butadien-1,2, bezogen auf das in der eingesetzten C₄-Kohlenwasserstofffraktion enthaltene Butadien-1,2, beträgt bei dieser Ausführungsform mindestens 85 %, bevorzugt mindestens 87 % und insbesondere mindestens 90 %. Die Ausbeute kann dabei noch weiter gesteigert werden, wenn man die Trennleistung der 2. Destillationskolonne verbessert, indem man beispielsweise die Zahl der theoretischen Böden erhöht, den Rücklauf vergrößert oder die Reinheitsansprüche für das Kopfprodukt Butadien-1,2 reduziert.

In einer dritten Ausführungsform des erfindungsgemäßen Verfahrens wird so vorgegangen, daß man in einer fraktionierten Destillation der Polymerisationsinhibitor-haltigen C₄-Kohlenwasserstofffraktion als Kopfprodukt alle niedriger als Butadien-1,2 siedenden C₄-Komponenten abtrennt, als Sumpfprodukt die C₅-Kohlenwasserstoffe und den Polymerisationsinhibitor erhält und als Seitenstrom Butadien-1,2 abnimmt. Die Abnahme dieses Seitenstroms erfolgt üblicherweise zwischen dem Zulauf des zu destillierenden C₄-Kohlenwasserstoffgemischs und dem Sumpf der Destillationkolonne.

Diese Gewinnung des reinen Butadien-1,2 aus dem C₄-Kohlenwasserstoffgemisch unter Verwendung nur einer Destillationskolonne kann besonders effektiv durchgeführt werden, wenn die Destillationskolonne zwischen Zulauf und Seitenstromentnahme des Butadien-1,2 als Trennblechkolonne ausgestaltet ist.

Das Sumpfprodukt dieser dritten Ausführungsform des erfindungsgemäßen Verfahrens enthält neben den C₅-Kohlenwasserstoffen und dem Polymerisationsinhibitor gegebenenfalls auch noch Restmengen des Butadien-1,2.

Gegebenenfalls ist es bei dieser dritten Ausführungsform zur Erzielung einer noch höheren Reinheit des Butadien-1,2 vorteilhaft, das als Seitenstrom entnommene Butadien-1,2 einer weiteren Destillation zu unterwerfen.

Auch bei dieser dritten Ausführungsform des erfindungsgemäßen Verfahrens können als Einbauten in der Destillationskolonne Böden, Füllkörper oder strukturierte Packungen Verwendung finden.

Bei allen drei Ausführungsformen des erfindungsgemäßen Verfahrens ist es von Vorteil, daß die jeweilige Fraktion, die nach der oder den fraktionierten Destillationen den Polymerisationsinhibitor enthält, noch gut pump- und transportfähig ist und keinen auskristallisierten Inhibitor enthält. Dies wäre bei den hohen Konzentrationen des Inhibitors in diesen Fraktionen zu erwarten, da die Löslichkeitsgrenze von beispielsweise TBC in Isopentan, welches eine Hauptkomponente in diesen Fraktionen ist, bei 20°C lediglich bei 0,5 % liegt und somit in den jeweiligen Fraktionen deutlich überschritten wird.

### Beispiel 1: Gewinnung von Butadien-1,2

Von 100 Gew.-Teilen eines C₄-Kohlenwasserstoff-Ausgangsgemischs, welches TBC als Polymerisationsinhibitor enthält, werden in der ersten Destillationskolonne mit ca. 90 theoretischen Trennstufen bei einem Rücklauf von ca. 100 Gew.-Teilen etwa 40 Gew.-Teile Kopfprodukt und 60 Gew.-Teile Sumpfprodukt erhalten, wobei das Kopfprodukt weniger als 1 Gew.-Teil Butadien-1,2 enthält. Die 60 Gew.-Teile des anfallenden Sumpfproduktes bestehen aus ca. 50 Gew.-Teilen Butadien-1,2 sowie aus ca. 10 Gew.-Teilen C₅-Kohlenwasserstoffen und des im Ausgangsgemisch vorhandenen TBC. Das Sumpfprodukt ist praktisch frei von sonstigen C₄-Kohlenwasserstoffen (Butane, Butene, Butadien-1,3 und C₄-Acetylene).

Das erhaltene erste Sumpfprodukt wird ohne weitere Reinigung in eine zweite Destillationskolonne mit ca. 45 theoretischen Trennstufen gefahren. Bei einem Rücklauf von ca. 250 Gew.-Teilen werden die 60 Gew.-Teile Sumpfprodukt aus der ersten Kolonne destillativ aufgetrennt in 45 Gew.-Teile eines zweiten Kopfprodukts sowie 15 Gew.-Teile eines zweiten Sumpfprodukts. Das Kopfprodukt der zweiten Destillationsstufe besteht aus nahezu reinem Butadien-1,2 (Reinheit > 99 Gew.%). Im Sumpfprodukt der zweiten Destillation befinden sich die C₅-Kohlenwasserstoffe, die gesamte Menge des Polymerisationsinhibitors TBC sowie etwa 5 Gew.-Teile Butadien-1,2.

## Patentansprüche

1. Verfahren zur Gewinnung von Butadien-1,2, bei dem eine Polymerisationsinhibitor-haltige C₄-Kohlenwasserstofffraktion mindestens einer fraktionierten Destillation unterworfen und dabei das Butadien-1,2 isoliert wird.

2. Verfahren nach Anspruch 1, wobei die eingesetzte Polymerisationsinhibitor-haltige C₄-Kohlenwasserstofffraktion einen Siedebereich von -15 bis +45°C aufweist.

3. Verfahren nach Anspruch 1, wobei es sich bei dem Polymerisationsinhibitor um 4-tert.-Butylbrenzcatechin handelt.

4. Verfahren nach Anspruch 1, wobei die Polymersationsinhibitor-haltige C₄-Kohlenwasserstofffraktion
| | |
|---|---|
| 0-5 Gew.%, bevorzugt 0-1 Gew.% | gesättigte C₄-Kohlenwasserstoffe, |
| 5-30 Gew.%, bevorzugt 10-20 Gew.% | Butene, |
| 10-55 Gew.%, bevorzugt 20-55 Gew.% | Butadien-1,3, |
| 0,1-2 Gew.%, bevorzugt 0.1-1 Gew.% | C₄-Acetylene, |
| 20-65 Gew.%, bevorzugt 30-65 Gew.% | Butadien-1,2, |
| 5-20 Gew.%, bevorzugt 5-10 Gew.% | C₅-Kohlenwasserstoffe sowie |
| 0.2-2 Gew.%, bevorzugt 0.1-1 Gew.% | Polymerisationsinhibitor |
enthält.

5. Verfahren nach Anspruch 1, **dadurch gekennzeichnet, daß** man
a) in einer ersten fraktionierten Destillation als erstes Kopfprodukt die leichtsiedenden C₄-Kohlenwasserstoffe und als erstes Sumpfprodukt eine Fraktion, die Butadien-1,2, die C₅-Kohlenwasserstoffe und den Polymerisationsinhibitor enthält, entnimmt, und
b) das erste Sumpfprodukt einer zweiten fraktionierten Destillation zuführt und dort als zweites Kopfprodukt das Butadien-1,2 gewinnt und als zweites Sumpfprodukt die C₅-Kohlenwasserstoffe und den Polymerisationsinhibitor erhält.

6. Verfahren nach Anspruch 1, **dadurch gekennzeichnet, daß** man
a) in einer ersten fraktionierten Destillation als erstes Kopfprodukt die leichtsiedenden C₄-Kohlenwasserstoffe und Butadien-1,2 und als erstes Sumpfprodukt die C₅-Kohlenwasserstoffe und der Polymerisationsinhibitor entnimmt und
b) das erste Kopfprodukt in eine zweite fraktionierte Destillation einbringt und dort als zweites Kopfprodukt die leichtsiedenden C₄-Kohlenwasserstoffe und als zweites Sumpfprodukt das Butadien-1,2 gewinnt.

7. Verfahren nach Anspruch 6, **dadurch gekennzeichnet, daß** man das Butadien-1,2 nicht als zweites Sumpfprodukt gewinnt, sondern bereits oberhalb des zweiten Sumpfes als Seitenstrom entnimmt.

8. Verfahren nach Anspruch 1, **dadurch gekennzeichnet, daß** man in der fraktionierten Destillation der Polymerisationsinhibitor-haltigen C₄-Kohlenwasserstofffraktion als Kopfprodukt alle niedriger als Butadien-1,2 siedenden C₄-Kohlenwasserstoffe abtrennt, als Sumpfprodukt die C₅-Kohlenwasserstoffe und den Polymerisationsinhibitor erhält und als Seitenstrom Butadien-1,2 abnimmt.

9. Verfahren nach Anspruch 8, wobei der Seitenstrom zwischen dem Zulauf der Polymerisationsinhibitor-haltigen C₄-Kohlenwasserstofffraktion und dem Sumpf abgenommen wird und die Destillationskolonne in diesem Bereich als Trennblechkolonne ausgestaltet ist.

10. Verfahren nach Anspruch 8, **dadurch gekennzeichnet, daß** man das als Seitenstrom entommene Butadien-1,2 einer weiteren Destillation unterwirft.

## Claims

1. A process for obtaining butadiene-1,2 in which a C₄ hydrocarbon fraction containing a polymerization inhibitor is subjected to at least one fractionated distillation to isolate the butadiene-1,2.

2. The process according to claim 1, wherein the employed C₄ hydrocarbon fraction containing a polymerization inhibitor has a boiling range of from -15°C to +45 °C.

3. The process according to claim 1, wherein the polymerization inhibitor is 4-tert.-butylcatechol.

4. The process according to claim 1, wherein said C₄ hydrocarbon fraction containing the polymerization inhibitor contains:
from 0 to 5%, preferably from 0 to 1%, by weight of saturated C₄ hydrocarbons;
from 5 to 30%, preferably from 10 to 20%, by weight of butenes;
from 10 to 55%, preferably from 20 to 55%, by weight of butadiene-1,3;
from 0.1 to 2%, preferably from 0.1 to 1%, by weight of C₄-acetylenes;
from 20 to 65%, preferably from 30 to 65%, by weight of butadiene-1,2;
from 5 to 20%, preferably from 5 to 10%, by weight of C₅ hydrocarbons; and
from 0.2 to 2%, preferably from 0.1 to 1%, by weight of polymerization inhibitor.

5. The process according to claim 1, **characterized in that**
a) in a first fractionated distillation, the low-boiling C₄ hydrocarbons are withdrawn as the first overhead product, and a fraction containing butadiene-1,2, the C₅ hydrocarbons and the polymerization inhibitor is withdrawn as the first bottom product; and
b) the first bottom product is subjected to a second fractionated distillation to obtain the butadiene-1,2 as the second overhead product, and the C₅ hydrocarbons and the polymerization inhibitor as the second bottom product.

6. The process according to claim 1, **characterized in that**
a) in a first fractionated distillation, the low-boiling C₄ hydrocarbons and butadiene-1,2 are withdrawn as the first overhead product, and the C₅ hydrocarbons and the polymerization inhibitor are withdrawn as the first bottom product; and
b) the first overhead product is subjected to a second fractionated distillation to obtain the low-boiling C₄ hydrocarbons as the second overhead product, and butadiene-1,2 as the second bottom product.

7. The process according to claim 6, **characterized in that** the butadiene-1,2 is not recovered as a second bottom product, but already withdrawn as a side stream above the second bottoms.

8. The process according to claim 1, **characterized in that** in a fractionated distillation of the C₄ hydrocarbon fraction containing the polymerization inhibitor, all C₄ components which are lower boiling than butadiene-1,2 are separated off as an overhead product, the C₅ hydrocarbons and the polymerization inhibitor are obtained as a bottom product, and butadiene-1,2 is withdrawn as a side stream.

9. The process according to claim 8, wherein the side stream is withdrawn between the inlet of the C₄ hydrocarbon fraction containing the polymerization inhibitor and the bottoms, and the distillation column the distillation column is designed as a baffle column in this region.

10. The process according to claim 8, **characterized in that** the butadiene-1,2 withdrawn as a side stream is subjected to a further distillation.

## Revendications

1. Procédé pour l'extraction de butadiène-1,2, dans lequel une fraction d'hydrocarbure en C₄ contenant un inhibiteur de polymérisation est soumise à au moins une distillation fractionnée au cours de laquelle le butadiène-1,2 est isolé.

2. Procédé selon la revendication 1, dans lequel la fraction d'hydrocarbure en C₄ contenant un inhibiteur de polymérisation utilisée a une plage d'ébullition comprise entre - 15 °C et + 45 °C.

3. Procédé selon la revendication 1, dans lequel l'inhibiteur de polymérisation est le 4-tertiobutylpyrocatéchol.

4. Procédé selon la revendication 1, dans lequel la fraction d'hydrocarbures en C₄ contenant un inhibiteur de polymérisation contient
| | |
|---|---|
| 0 - 5 | % en poids, de préférence 0 - 1 % en poids d'hydrocarbures en C₄ saturés, |
| 5 - 30 | % en poids, de préférence 10 - 20 % en poids de butène, |
| 10 - 55 | % en poids, de préférence 20 - 55 % en poids de butadiène-1,3, |
| 0,1 - 2 | % en poids, de préférence 0,1 - 1 % en poids d'acétylène en C₄, |
| 20 - 65 | % en poids, de préférence 30 - 65 % en poids de butadiène-1,2, |
| 5 - 20 | % en poids, de préférence 5 - 10 % en poids d'hydrocarbures en C₅ ainsi que |
| 0,2 - 2 | % en poids, de préférence 0,1 - 1 % en poids d'inhibiteur de polymérisation. |

5. Procédé selon la revendication 1, **caractérisé en ce que**
a) au cours d'une première distillation fractionnée, on prélève comme premier produit de tête les hydrocarbures en C₄ de bas point d'ébullition et comme premier produit de queue une fraction qui contient du butadiène-1,2, les hydrocarbures en C₅ et l'inhibiteur de polymérisation, et
b) on soumet le premier produit de queue à une deuxième distillation fractionnée au cours de laquelle on extrait le butadiène-1,2 comme deuxième produit de tête et on obtient les hydrocarbures en C₅ et l'inhibiteur de polymérisation comme deuxième produit de queue.

6. Procédé selon la revendication 1, **caractérisé en ce que**
a) au cours d'une première distillation fractionnée, on prélève comme premier produit de tête les hydrocarbures en C₄ de bas point d'ébullition et le butadiène-1,2 et comme premier produit de queue les hydrocarbures en C₅ et l'inhibiteur de polymérisation, et
b) on soumet le premier produit de tête à une deuxième distillation fractionnée au cours de laquelle on extrait les hydrocarbures en C₄ de bas point d'ébullition comme deuxième produit de tête et le butadiène-1,2 comme deuxième produit de queue.

7. Procédé selon la revendication 6, **caractérisé en ce qu'**on extrait le butadiène-1,2 non pas comme deuxième produit de queue, mais déjà sous forme de flux latéral juste au-dessus du deuxième bas de la colonne.

8. Procédé selon la revendication 1, **caractérisé en ce qu'**au cours de la distillation fractionnée de la fraction d'hydrocarbures en C₄ contenant un inhibiteur de polymérisation, on sépare comme produit de tête tous les hydrocarbures en C₄ ayant un point d'ébullition inférieur à celui du butadiène-1,2, on obtient comme produit de queue les hydrocarbures en C₅ et l'inhibiteur de polymérisation et on enlève le butadiène-1,2 comme flux latéral.

9. Procédé selon la revendication 8, dans lequel le flux latéral est enlevé entre l'arrivée de la fraction d'hydrocarbures en C₄ contenant un inhibiteur de polymérisation et le bas de la colonne et la colonne de distillation dans cette zone est conçue comme une colonne avec tôles de séparation.

10. Procédé selon la revendication 8, **caractérisé en ce que** le butadiène-1,2 enlevé comme flux latéral est soumis à une autre distillation.
